# EUROPÄISCHE PATENTSCHRIFT

(11) **EP 1 339 664 B1**
(45) Veröffentlichungstag und Bekanntmachung des Hinweises auf die Patenterteilung: **03.10.2007**
(21) Anmeldenummer: 01990417.6
(22) Anmeldetag: 16.11.2001
(51) Int. Cl.: C07C 51/353, C07C 55/24, C07C 231/12, C07C 233/18, C25B 3/00, C25B 3/10, C07D 307/32, C07C 67/00

(54) **HERSTELLUNG VON BUTANTETRACARBONSÄUREDERIVATEN MITTELS GEKOPPELTER ELEKTROSYNTHESE**
PRODUCTION OF BUTANE TETRACARBOXYLIC ACID DERIVATIVES BY MEANS OF COUPLED ELECTROSYNTHESIS
PRODUCTION DE DERIVES D'ACIDE BUTANE-TETRACARBOXYLIQUE AU MOYEN D'UNE ELECTROSYNTHESE COUPLEE

(30) Priorität: 22.11.2000 DE 10057888
(43) Veröffentlichungstag der Anmeldung: 03.09.2003
(73) Patentinhaber: BASF Aktiengesellschaft, 67056 Ludwigshafen (DE)
(72) Erfinder: PÜTTER, Hermann, 67433 Neustadt (DE); WEIPER-IDELMANN, Andreas, 67165 Waldsee (DE)
(74) Vertreter: Isenbruck, Günter
(86) Internationale Anmeldenummer: PCT/EP2001/013319
(87) Internationale Veröffentlichungsnummer: WO 2002/042249

(56) Entgegenhaltungen:
- EP-A- 0 433 260
- FR-A- 2 597 509
- US-A- 6 063 256
- DATABASE WPI Section Ch, Week 199329 Derwent Publications Ltd., London, GB; Class A41, AN 1993-232823 XP002195571 & JP 05 156478 A (MITSUI TOATSU CHEM INC), 22. Juni 1993 (1993-06-22)
- DATABASE WPI Section Ch, Week 197841 Derwent Publications Ltd., London, GB; Class A41, AN 1978-73560A XP002195572 & JP 53 101311 A (MITSUBISHI CHEM IND LTD) , 4. September 1978 (1978-09-04)
- LI, W.; NONAKA, T.; CHOU, T.-S.: "Paired Electrosynthesis of Organic Compounds" ELECTROCHEMISTRY, Bd. 67, Nr. 1, 1998, Seiten 4-10, XP001064658 in der Anmeldung erwähnt

## Beschreibung

Die vorliegende Erfindung betrifft ein Verfahren zur Herstellung von Butantetracarbonsäurederivaten mittels gekoppelten Elektrosynthese sowie deren Verwendung.

Butantetracarbonsäurederivate sind derzeit als wichtige Zwischen- oder Vorprodukte beispielsweise in der kunststoffverarbeitenden Industrie von Interesse. Demgemäß wächst auch der Wunsch nach einem Verfahren, durch welches Butantetracarbonsäurederivate in ausreichender Menge sowie gleichzeitig in der für die Weiterverarbeitung notwendigen Reinheit hergestellt werden können. Im Zuge der Zeit ist es ebenfalls wünschenswert, daß derartige Herstellungsverfahren ressourcenschonend und kostengünstig sind.

Nach einer langen Entwicklungsphase stellt die präparative organische Elektrochemie seit etwa den siebziger Jahren einen Weg dar, um Produkte umweltfreundlich und mit hoher Selektivität herzustellen. Bisher wurde diese Präparationstechnik bevorzugt zur "Monosynthese" von Substanzen entweder durch anodische Oxidation oder kathodische Reduktion genutzt. Das jeweilige an der Gegenelektrode anfallende Koppelprodukt muß in der Regel abgetrennt und entsorgt werden. Selbst wenn dies problemlos gelingt, ist der für die Koppelreaktion notwendige Energieaufwand vergeblich aufgebracht worden. Dies manifestiert sich in einem hohen Stromverbrauch derartiger elektrochemischen Monosynthesen, da die Gegenreaktion sozusagen wirtschaftlich ungenutzt bleibt. Weiterhin verursachen die dabei entstehenden Nebenprodukte als Abfallstoffe hohen Entsorgungskosten, welche oft nicht mehr im wirtschaftlichen Verhältnis zu dem gewonnenen Produkt stehen.

Nachteilig daran ist weiterhin unter anderem ein zwangsläufig mit der Verwendung von geteilten Elektrolysezellen verbundener hoher apparativer Aufwand, da zwei Zellkreise erforderlich sind, welche durch eine Membran oder ein Diaphragma getrennt werden müssen. Diese Trennung führt aufgrund der dadurch auftretenden ohmsche Wärme zu einem Verlust an Energie. Um diesen Energieverlust zu minimieren, beschickt man häufig den Kreislauf für die Gegenelektrode mit einer wäßrigen (> 80% H₂O) Leitsalzlösung. Daraus entsteht der Nachteil, daß als Kathodenprodukt fast ausschließlich Wasserstoff (oder als Anodenprodukt weitgehend Sauerstoff) erzeugt wird, welcher, da verunreinigt, als Abfallprodukt durch Verbrennung entsorgt werden muß.

So ist auch zur Herstellung von Butantetracarbonsäureestern aus der EP-A 0 433 260 ausschließlich ein elektrochemisches Verfahren zu dessen Monosynthese bekannt. Hierbei wird Maleinsäuredialkylester zu Butantetracarbonsäureestern umgesetzt und anschließend weiter zu Butantetracarbonsäure hydrolysiert. Bei dieser Umsetzung handelt es sich um eine kathodische Elektrodimerisierung, welche in einer ungeteilten elektrochemischen Zelle unter Zusatz u.a. eines Alkohols stattfindet. Der Alkohol, in der Regel Methanol, wird dabei an der Anode zu Oxidationsprodukten, wie beispeilsweise Methoxymethanol oder Methylformiat umgesetzt. Im Anschluß an die Elektrodimerisierung muß der jeweilige Butantetracarbonsäureester durch Kristallisation und anschließender Filtration von der Elektrolyselösung abgetrennt werden und kann in einem weiteren Schritt im sauren Medium zu Butantetracarbonsäure hydrolysiert werden.

Nachteilig an diesem Verfahren ist zum einen, daß es sich dabei um eine Monosynthese verbunden mit den oben aufgeführten Nachteilen dieser Synthesearten wie beispielsweise der Entsorgung der im Laufe der Elektrolyse an der Anode entstehenden Produkte wie Methoxymethanol als unerwünschtes Abfallprodukt handelt.

Somit müssen nicht nur die Entsorgungskosten der Abfallprodukte, sondern auch die gesamten Kosten hervorgerufen durch den apparativen, energetischen sowie stofflichen Einsatz zur Herstellung des nur einen Produkts durch dessen wirtschaftliche Verwertung zu tragen sein, da die bei seiner Herstellung an der Anode parallel dazu entstehenden Produkte bisher nicht wirtschaftlich verwertbar sind.

Bezüglich einer möglichen Kopplung von Anoden- und Kathodenreaktion wurden bisher in der Literatur, beispielsweise in M.M. Baizer et al. ,"Organic Electrochemistry", M. Dekker, New York 1991, Seiten 1422ff. oder W. Li, T. Nonaka, T.-S. Chou, Electrochemistry 67, 1 (1998) Seite 4 bis 10 immer wieder Vorschläge zu einer derartigen Kopplung von Anoden- und Kathodenreaktion gemacht, um einige der oben genannten Nachteile zu vermeiden. Diese Verfahren weisen jedoch weitere Nachteile auf, welche eine Umsetzung vom Labor- in den technischen Maßstab bisher unmöglich gemacht haben. So verhindert beispielsweise die auftretende Vergiftung wenigstens einer Elektrode sowie der Verlust der Selektivität im Laufe des Verfahrens der dort beschriebenen gekoppelten Elektrosynthesen regelmäßig ihre Umsetzung in einen wirtschaftlich nutzbaren Rahmen.

Nachteilig bei diesen Verfahren sind die häufig auftretenden Reaktionen der beiden an den Elektroden entstehenden Produkte oder sogar schon die Reaktion der Edukte miteinander. Dadurch wird zum einen die Ausbeute des eigentlichen Hauptprodukts aufgrund der dadurch entstehenden weiteren Nebenprodukte stark gesenkt, als auch die Aufarbeitung der Reaktionslösung stark erschwert.

Oft führen die eben aufgeführten Nachteile zu technisch nicht umsetzbaren Zellkonstruktionen oder so geringen Ausbeuten, daß das Verfahren aufgrund seiner Ineffizienz keinen ökonomischen Fortschritt mehr darstellt.

Erst in jüngster Zeit wurde die gekoppelte Elektrosynthese erfolgreich wirtschaftlich angewandt. So wurde ein Verfahren entwickelt und patentiert (DE-A1 196 18 854), welches sich zur Herstellung von Phthalid, koppelbar mit einer Vielzahl an anodischen Oxidationen, welche ihrerseits zu einem weiteren ebenfalls wirtschaftlich verwertbaren Produkt führen, eignet. Jedoch ist bisher kein weiterer scher Partner für ein derartiges im technischen Maßstab realisierbares Verfahren bekannt.

Eine für eine nachhaltige Entwicklung auf diesem technischen Gebiet drängende Frage ist deshalb, ob es aus der wissenschaftlichen Literatur bekannte kathodische Reaktionen gibt, die ebenfalls unempfindlich gegen Anodenprozesse sind und damit den Resourcenbedarf der bisherigen, technisch realisierbaren Elektrosynthesen senken können.

Demgemäß lag der Erfindung die Aufgabe zugrunde ein Verfahren bereitzustellen, durch welches Butantetracarbonsäurederivate unter Vermeidung der oben angesprochenen Nachteile wirtschaftlich hergestellt werden können.

Es wurde überraschend gefunden, daß Butantetracarbonsäurederivate vorteilhaft erhalten werden können, indem deren elektrolytische Darstellung an der Kathode mit geeigneten anodischen Prozessen gekoppelt wird. Außerdem wurde gefunden, daß die Zahl der möglichen Kopplungspartner überraschend hoch ist.

Gegenstand der vorliegenden Erfindung ist somit ein Verfahren zur Herstellung von Butantetracarbonsäurederivaten als Wertprodukt I durch gekoppelte Elektrosynthese, wobei eine Verbindung ausgewählt aus der Gruppe bestehend aus Maleinsäureestern, Maleinsäureesterderivaten, Fumarsäureestern oder Fumarsäureesterderivaten, bei welchen wenigstens ein Wasserstoffatom der Positionen 2 und 3 durch inerte Gruppen substituiert sein kann, kathodisch reduziert wird und an der Anode ein Wertprodukt II erhalten wird, welches sich dadurch kennzeichnet, dass als Wertprodukt II ein Produkt ausgewählt wird aus einer Gruppe bestehend aus Acetalen aromatischer Aldehyde, methoxylierten Heterocyclen, Aromaten und Olefinen, methoxylierten Amiden sowie α-Hydroxyketalen, α-Hydroxyacetalen, Carbonsäuren und Carbonsäureestern, und im Elektrodenmaterial Materialien auf Kohlenstoffbasis enthalten sind.

Unter dem Begriff "gekoppelte Elektrosynthese" wird im Rahmen der vorliegenden Erfindung ein Verfahren zur Herstellung von bevorzugt organischen Präparaten verstanden. Dieses zeichnet sich dadurch aus, daß die kathodische Reduktion durch welche ein Wertprodukt I erzeugt wird, mit einer an der Anode ablaufenden Oxidation welche zu einem Wertprodukt II führt, gekoppelt ist. Durch diese Verfahrensführung läßt sich der eingesetzte Strom "zweifach" nutzten. Dies führt im Vergleich zu den bisher herkömmlich eingesetzten Monosynthesen der Wertproduk-te I und II zu einem verbesserten wirtschaftlichen Verhältnis von Gesamtproduktausbeute zu dafür aufzuwendender Energie.

Die Elektrolyse kann in der Regel in geteilten oder ungeteilten Zellen durchgeführt werden.

In einer bevorzugten Ausführungsform der vorliegenden Erfindung wird die gekoppelte Elektrosynthese zur Herstellung von Butantetracarbonsäurederivaten in einer ungeteilten elektrochemischen Zelle durchgeführt. Dadurch werden die oben beschriebenen Nachteile, welche bei Elektrolysen in geteilten Zellen häufig auftreten, weitgehend vermieden.

Als Edukte zur Synthese von Butantetracarbonsäurederivaten mittels der gekoppelten Elektrosynthese können grundsätzlich alle Maleinsäure- oder Fumarsäureester eingesetzt werden. Vorzugsweise werden C₁-C₆ -Alkylester der genannten Säuren, besonders bevorzugt Malein- oder Fumarsäuredimethylester eingesetzt.

Weiterhin können als Edukte auch Maleinsäure- oder Fumarsäureesterderivate eingesetzt werden, in welchen wenigstens ein Wasserstoffatom der Positionen 2 und 3 durch inerte Gruppen substituiert ist. Die einzusetzenden Maleinsäure- oder Fumarsäureesterderivate können im Rahmen der vorliegenden Erfindung beispielsweise Methyl-, Cyano-, Hydroxymethyl- sowie Methoxy- substituierte Derivate sein.

Ein weitere Ausführungsbeispiel der vorliegenden Erfindung ist dadurch gekennzeichnet, daß das Malein- oder Fumarsäureesterderivat Maleinsäure- oder Fumarsäuredimethylester ist.

Als Elektrodenmaterial für die in der vorliegenden Erfindung verwendbaren Elektroden können im Prinzip alle für die präparative organische Elektrochemie gängigen Elektrodenmaterialien verwendet werden.

Die erfindungsgemäße Umsetzung kann in ungeteilten und geteilten Zellen durchgeführt werden. Vorzugsweise wird in ungeteilten Zellen gearbeitet.

### Beispielhaft genannt sei folgende Apparatevariante:

Ungeteilte Zellen mit planparalleler Elektrodenanordnung oder kerzenförmigen Elektroden kommen bevorzugt dann zum Einsatz, wenn weder Edukte noch Produkte , die an Anode oder Kathode hergestellt oder umgesetzt werden, in störender Weise durch den jeweils anderen Elektrodenprozeß verändert werden oder miteinander reagieren. Vorzugsweise werden die Elektroden planparallel angeordnet, weil bei dieser Ausführungsform bei kleinem Elektrodenspalt (0,5 mm bis 30 mm, vorzugsweise 1 bis 10 mm) eine homogene Stromverteilung gegeben ist. Vorzugsweise können in diesem Fall die Elektroden einzeln oder zu mehreren gestapelt angewendet werden. Im letzteren Fall handelt es sich um die Verwendung von sogenannten Stapelelektroden, die in der sogenannten Plattenstapelzelle seriell bipolar geschaltet werden können.

Ferner können auch Zellen eingesetzt werden, wie sie in der DE-A 195 33 773 beschreiben sind.

In einem Ausführungsbeispiel der vorliegenden Erfindung sind im Elektrodenmaterial Materialien auf Kohlenstoffbasis und metallische Werkstoffe einzeln oder als Gemisch aus zwei oder mehr davon enthalten. Bevorzugt werden Elektroden aus auf Kohlenstoff basierenden Materialien wie Graphit, glassy Carbon, Graphitfilz oder Elektroden aus Gerätegraphit eingesetzt.

Die im Rahmen der Erfindung eingesetzten Elektrolyte bestehen in der Regel aus den im Lösemittel oder Lösemittelgemisch gelösten Edukten sowie einem Leitsalz. Bei Verwendung der in der DE-A 195 33 773 beschriebenen Plattenstapelzellen kann auf den Einsatz von Leitsalz verzichtet werden, indem die in D. Hoormann et al GDCh-Monografie 14, "Elektrochemische Reaktionstechnik und Synthese" J. Russow, G. Sandstede, R. Staab (Hrsg.), GDCh, 1999, S. 537 - 543 und V. Kröner et al, ibid., S. 484 - 490 beschriebenen Zellkonstruktion verwendet wird.

In einer Ausführungsform der Erfindung liegt die Gesamtkonzentration von Edukten und Produkten im Elektrolyten jeweils im Bereich von 1 bis 70 Gew.-%, bevorzugt im Bereich von 3 bis 50 Gew.-%, besonders bevorzugt im Bereich von 10 bis 40 Gew.-%, wobei ihre Summe 75 Gew.-% nicht übersteigen sollte.

Als Lösemittel für die eingesetzten Edukte können alle in der organischen Elektrochemie üblichen Lösemittel sowie auch Mischungen aus zwei oder mehr davon verwendet werden.

In einer bevorzugten Ausführungsform der vorliegenden Erfindung wird bei der Durchführung des erfindungsgemäßen Verfahrens ein Lösemittel oder ein Lösemittelgemisch eingesetzt, wobei das Lösemittel oder das Lösemittelgemisch einen Gewichtsanteil von größer oder gleich 50 %, bevorzugt größer oder gleich 65 %, bezogen auf das Gesamtgewicht aller bei dem Verfahren eingesetzten Substanzen aufweist.

In einem Ausführungsbeispiel der vorliegenden Erfindung werden Lösemittel oder Lösemittelgemisch ausgewählt aus einer Gruppe bestehend aus Methanol, Ethanol, Essigsäure, Dimethylsulfoxid, Tetrahydrofuran, Dioxan, Acetonitril und Gemischen aus zwei oder mehr davon eingesetzt.

Im Rahmen einer Ausführungsform der vorliegenden Erfindung wird das bevorzugt Lösemittel oder Lösemittelgemisch ausgewählt aus einer Gruppe bestehend aus aliphatischen C₁- bis C₉ - Alkoholen, bevorzugt C₁- bis C₄ - Alkoholen. Besonders wird als Lösemittel jedoch Methanol eingesetzt.

Im Rahmen der vorliegenden Erfindung enthält das gewählte Lösemittel oder Lösemittelgemisch in der Regel weniger als 10 Gew.-% Wasser, bevorzugt weniger als 5 Gew.-% Wasser, besonders bevorzugt weniger als 1 Gew.-% Wasser.

Als Leitsalze können im Rahmen der vorliegenden Erfindung alle in der präparativen Elektrochemie verwendbaren Leitsalze oder auch Mischungen aus zwei oder mehr davon eingesetzt werden.

Demgemäß wird in einer Ausführungsform der vorliegenden Erfindung zur Durchführung des erfindungsgemäßen Verfahrens mindestens ein Leitsalz eingesetzt.

Dieses wenigstens eine Leitsalz wird ausgewählt aus einer Gruppe bestehend aus Tetraalkylammoniumsalzen, Tetrafluorboraten, Alkalimetallsalzen, Salzen von aromatisch substituierten Sulfonsäuren, Salzen der Methansulfonsäure, Salzen der Perchlorsäure, Bromiden, Iodiden, Phosphaten, Phosphonaten, Alkoxycarbonaten, Carboxylaten, Sulfaten, Alkylsulfonaten sowie Alkylsulfaten, insbesondere Acetaten und Formiaten sowie Salzen der Maleinsäure oder Fumarsäure oder deren Halbester.

Bevorzugt werden im Rahmen der vorliegenden Erfindung als Leitsalz Salze der Methansulfonsäure, sowie der Essigsäure, besonders bevorzugt werden Salze der Methylschwefelsäure eingesetzt.

Im Rahmen der vorliegenden Erfindung wird das wenigstens eine Leitsalz in einer Menge von 0,2 bis 15 Gew.-%, bevorzugt in einer Menge von 0,3 bis 5 Gew.-%, besonders bevorzugt in einer Menge von 0,4 bis 3 Gew.-% bezogen auf den Elektrolyten eingesetzt.

In einer weiteren Ausführungsform wird das Leitsalz durch eine Ionenaustauschermembran ersetzt.

In der Regel ist die gekoppelte Elektrosynthese bei jeder, mit den eingesetzten Substanzen sowie deren Lösemitteln vereinbaren Temperatur durchführbar. Im Rahmen der vorliegenden Erfindung wird die Elektrolyse bei Temperaturen im Bereich von 0° bis zum Siedepunkt des jeweilig verwendeten Lösemittels oder Lösemittelgemischs, bevorzugt jedoch bei Temperaturen im Bereich von 0° bis 100°C, besonders bevorzugt bei Temperaturen im Bereich von 25° bis 65°C durchgeführt.

Prinzipiell kann das im anodischen Kopplungsprozess hergestellte Wertprodukt II unabhängig von dem kathodisch hergestellten Wertprodukt I gewählt werden. Dadurch ist es möglich, daß sich die Produktion flexibel der wirtschaftlichen Nachfrage für beide Wertprodukte anpassen läßt.

Das erfindungsgemäße Verfahren ist somit nicht mit den sonst üblichen Nachteilen von Kopplungsprozessen, wie beispielsweise der starren Mengenrelation beider Kopplungsprodukte zueinander, behaftet, welche die Marktflexibilität eingeschränkt.

Gemäß der vorliegenden Erfindung wird als Wertprodukt II ein Produkt erhalten, welches ausgewählt wird aus einer Gruppe bestehend aus Acetalen aromatischer Aldehyde, methoxylierten Heterocyclen, Aromaten und Olefinen, methoxylierten Amiden sowie α-Hydroxyketalen und α-Hydroxyacetalen, Carbonsäuren und Carbonsäureestern.

Als anodische Kopplungsreaktionen eignen sich überraschender Weise weitgehend alle dem Fachmann allgemein bekannten elektrochemischen Oxidationsreaktionen, wie beispielsweise die Oxidationen von C-O- oder C-N-Funktionen. Auch die Bildung von Heterocyclen, wie die Bildung von Oxazolidinonen aus den entsprechenden N-(Hydroxyethyl)-formamiden sowie die Bildung von entsprechenden sechs- und siebengliedrigen Ringen ist als anodische Kopplungsreaktion geeignet. Des weiteren geeignet sind auch viele aus Lehrbüchern der organischen Elektrochemie, wie beispielsweise D. Kyriakou, Modem E-organic Chemistry, Springer, Berlin 1994, Kapitel 4.2 bekannten anodische Reaktionen wie Alkoxylierung, Acyloxylierungen sowie die Kupplung von Olefinen wie z.B. Enolethern. Auch die Dimerisierung von CH-aktivierten Verbindungen oder Aromaten, wie Trimethylbenzol oder die Oxidation von Aminen, Alkoholen oder von aromatischen Systemen, z.B. Hydrochinonethern oder Heterocyclen wie z.B. Furanen können als mögliche anodische Kopplungsreaktion gewählt werden.

Einige der anodischen Koppelprozesse werden bevorzugt in Anwesenheit eines Mediators durchgeführt. Mögliche anodische Koppelprozesse und deren Mediatisierung werden beispielsweise in D. Kyriakou, Modem Electroorganic Chemistry, Springer, Berlin 1994, in Kapitel 4.2 beschrieben. Als Mediatoren eigenen sich insbesondere Halogenverbindungen, vor allem Bromide oder Iodide.

Weiterhin ist es im Rahmen der vorliegenden Erfindung möglich, daß das zur anodischen Herstellung von Wertprodukt II eingesetzte Edukt mit dem wenigstens einen Lösemittel reagiert, obwohl die Menge des Lösemittels, welche für die anodische Reaktion benötigt wird, gegenüber der anodischen Monofahrweise deutlich durch Gegenwart der kathodischen Reaktionsteilnehmer vermindert wird.

Im Rahmen eines bevorzugten Ausführungsbeispiels der vorliegenden Erfindung wird die anodische Kopplungsreaktion so gewählt, daß sich der Siedepunkt von Wertprodukt I und Wertprodukt II um eine Temperaturdifferenz von gleich oder größer 10°C unterscheiden, so daß die Trennung beider Wertprodukte bei der Aufarbeitung der Elektrolyselösung beispielsweise durch destillative Trennung problemlos möglich ist.

Die Art der Aufarbeitung der Elektrolyselösung richtet sich im allgemeinen nach den Eigenschaften der hergestellten Wertprodukten I und II. In der Regel kann die Aufarbeitung und Trennung der Wertprodukte über alle dem Fachmann gängigen Aufarbeitungsmethoden erfolgen, wie beispielsweise Destillation, Umkristallisation oder auch Fällungsreaktionen.

Im Rahmen der vorliegenden Erfindung werden die entsprechenden Butantetracarbonsäurederivate als Wertprodukt I in der Regel von der übrigen Elektrolyselösung abdestilliert. Überraschenderweise wurde hierbei festgestellt, daß die vier Carbonsäureesterfunktionen der erfindungsgemäß hergestellten Butantetracarbonsäurederivate auch unter den thermisch drastischen Bedingungen einer Destillation nicht mit den kathodischen Produkten reagieren, obwohl im allgemeinen sowohl die Esterfunktion des Edukts, als auch die des Produkts, beispielsweise auch schon unter Elektrolysebedingungen leicht zu derartigen Reaktionen neigen.

In einer weiteren Ausführungsform des vorliegenden erfindungsgemäßen Verfahrens wird zunächst das Lösemittel oder das Lösemittelgemisch sowie alle weitere niedrigsiedenden Bestandteile des Elektrolyseaustrages abdestilliert, wobei das Lösemittel oder das Lösemittelgemisch ganz oder teilweise dem Elektrolysekreislauf wieder zugeführt werden kann. Anschließend wird das zurückbleibende Gemisch, welches vorwiegend die beiden gewünschten Produkte enthält, in einer Destillationskolonne mit weniger als 5 theoretischen Böden, zur Abtrennung eines Produktes im Vakuum destilliert. Bevorzugt werden hierbei Dünnschichtverdampfer sowie Sambays eingesetzt.

Nach dem erfindungsgemäßen Verfahren erhält man die gewünschten Butantetracarbonsäurederivate in hohen Ausbeuten und Reinheit. Gleichzeitig wird an der Anode ein weiteres wirtschaftlich verwertbares Wertprodukt II hergestellt, ohne daß die Strom- und Materialausbeute an der Kathode dadurch merklich beeinträchtigt wird.

Prinzipiell kann jede dem Fachmann bekannte geteilte oder ungeteilte, bevorzugt jedoch eine ungeteilte Elektrolysezelle zur Durchführung des erfindungsgemäßen Verfahrens verwendet werden. Die Elektrolysezelle kann auch Bestandteil einer Umlaufapparatur sein, in welcher der jeweilig verwendete Elektrolyt umgepumpt, geheizt oder auch gekühlt werden kann. Der im Rahmen der vorliegenden Erfindung bevorzugte Aufbau der Elektrolysezelle wird in einem unten aufgeführten Beispiel näher erläutert. dung bevorzugte Aufbau der Elektrolysezelle wird in einem unten aufgeführten Beispiel näher erläutert.
Da das erfindungsgemäße Verfahren mit den oben aufgeführten Vorteilen wie flexibler Produktkoordinierung, doppelter Energieausnutzung usw. gekoppelt ist, eignet es sich bevorzugt für einen Einsatz in der chemischen sowie pharmazeutischen Industrie.

Die vorliegende Erfindung soll nunmehr anhand von ausgewählten Beispielen näher erläutert werden.

### Beispiele

### Beispiel 1: Elektrolysezelle

In einer ungeteilten Zelle sind 11 Ringscheibenelektroden von einer Fläche von jeweils etwa 140 cm² so angeordnet, daß sie einen Stapel bilden. Die Scheiben sind jeweils etwa 50 mm stark. Durch Abstandshalter wird der Abstand zwischen den Scheiben auf etwa 1 mm eingestellt, so daß 10 Spalten zwischen den 11 Ringscheibenelektroden entstehen. Als Elektrodenmaterial wird Graphit verwendet. Die inneren Scheiben sind während der Elektrolyse bipolar geschaltet. Die oberste Ringscheibenelektrode ist mit Hilfe eines Graphitstempels sowie einer Deckscheibe anodisch kontaktiert. Die unterste Elektrode ist über die Bodenplatte der Elektrolysezelle kathodisch kontaktiert. Der jeweilige Elektrolyt wird durch die zentrale Bodenplatte in die Zelle eingebracht, verteilt sich sodann zwischen den Spalten der Elektroden und kann die Zelle über eine Öffnung oberhalb der obersten Elektrode wieder verlassen. Die eben beschriebene Zelle ist Bestandteil einer Umlaufapparatur.

### Beispiel 2: Elektrolyse von Maleinsäuredimethylester und p-Xylol

2152 g Maleinsäuredimethylester, 396 g p-Xylol, 209 g gesättigte Natriummonomethylsulfatlösung sowie 1243 g Methanol wurden in einer in Beispiel 1 beschriebenen Elektrolysezelle bei einer Temperatur von 48 °C, einer Stromstärke von 5 A elektrolysiert. Im Laufe der Elektrolyse nahm die Stromstärke von 5 auf 3,75 A ab. Die Spannung der Teilzellen liegt über den gesamten Elektrolysevorgang hindurch bei 6V. Nach einem Stromeinsatz von 1 F/Mol Maleinsäuredimethylester wurde die Elektrolyse abgebrochen.

Im Elektrolyseaustrag lagen, ermittelt über die GC-Flächenprozente, Butantetracarbonsäuremethylester und 2-Methoxy-bernsteinsäuredimethylester zu je 20 Gew.-% vor. p-Xylol sowie die beiden weiteren Methoxylierungsprodukte p-Tolylmethylether und p-Tolylaldehyddimethylacetal als Wertprodukt II lagen in einem Mengenverhältnis von 1 zu 1,8 zu 2, 4. Der p-Xylolumsatz lag bei größer 85 Gew.-%.

Die Elektrolyselösung wurde destillativ aufgearbeitet.

Butantetracarbonsäuretetramethylester wurde mit einer Ausbeute von 45 % bezogen auf den eingesetzten Maleinsäuredimethylester und einer Reinheit von größer 97 % erhalten.

Das Wertprodukt II p-Tolylaldehyddimethylacetal wird u.a. als Zwischenprodukt für die Herstellung von beispielsweise Pharmaka, Pflanzenschutzmittel, UV-Stabilisatoren sowie Trübungsverhinderer bei Kunststoffen eingesetzt.

### Beispiel 3: Elektrolyse von Maleinsäuredimethylester und Dimethylformamid

In einer Elektrolysezelle gemäß Beispiel 1 werden 600 g Maleinsäuredimethylester, 300 g Dimethylformamid und 15 g LiBF₄ in 2085 g Methanol bei einer Temperatur von 45 °C, einer Stromstärke von 5 A über einen Zeitraum von 3,35 h elektrolysiert. Nach dieser Zeit waren über 95 Gew.-% an Maleinsäuredimethylester umgesetzt. Als weiteres Wertprodukt II wurde N-Methoxymethyl-N-methylformamid aus Dimethylformamid gebildet.

Es wurden vier gleiche Elektrolyseansätze gefahren und gemeinsam destillativ aufgearbeitet.

Butantetracarbonsäuretetramethylester wurde mit einer Ausbeute von 60 Gew.-% bezogen auf den eingesetzten Maleinsäuredimethylester und einer Reinheit von größer 95 % erhalten.

N-Methoxymethyl-N-methylformamid wurde mit einer Ausbeute von über 80 Gew.-% bezogen auf das eingesetzte Dimethylformamid erhalten. Das Produkt wird vorwiegend bei Untersuchungen zur Methoxylierung von Amiden eingesetzt. Weiterhin wird es als Reagenz zur Einführung von N-CH₂-Funktionen, beispielsweise in aromatischen Systeme verwendet.

### Beispiel 4: Elektrolyse von Maleinsäuredimethylester und Furan

Die Elektrolysezelle beinhaltete in diesem Fall nur 3 Spalte von jeweils 0,5 mm. Die Kathodenseiten der Graphitelektroden waren jeweils mit einer 2 mm starken Lage aus Graphitfilz (KFD 02, Fa. SGL Carbon) versehen.

3500 g Elektrolyt mit 9,4% Furan, 40% Maleinsäuredimethylester, 1% NaBr und 49,6% Methanol wurde bei 19-24°C mit einer Stromstärke von 5 A bis zu einem Ladungseintrag von 1,1 F bezogen auf Maleinsäuredimethylester (entsprechend 2,4 F bezogen auf Furan) elektrolysiert. Der Austrag enthielt die Produkte Furan:Dimethoxyfurane im Verhältnis 1,0:9,3 entsprechend einem Umsatz von 90 %. Die Selektivität Dimethoxydihydrofuran/ Dimethoxytetrahydrofuran lag bei 99%. Maleinsäuredimethylester wurde zu über 99% umgesetzt. Butantetracarbonsäuretetramethylester und Bernsteinsäuredimethylester waren im Verhältnis 1:0,31 entstanden, Butantetracarbonsäuretetramethylester und Methoxybemsteinsäure im Verhältnis 1: 1,15.

### Beispiel 5: Elektrolyse von Maleinsäuredimethylester und DMF

In einer Zelle, wie sie in L.Kröner et al. GDCh-Monographie 14, "Elektrochemische Reaktionstechnik und Synthese" J. Russow, G. Sandstede, R. Staab Hrsg., GDCh, Frankfurt 1999, Seite 484-490 beschrieben wird, wurde elektroysiert. Sieben 6 mm starke Graphitfilzringscheiben mit einer Fläche von jeweils 0,61 dm² wurden alternierend mit 7 Nafionmembranen der gleichen Fläche zu einem Stapel vereinigt. Die oberste Filzscheibe wurde durch eine Graphitscheibe anodisch kontaktiert, die untere entsprechend kathodisch kontaktiert.

Die Nafionmembranen (Nafion 117, DuPont) wurden vor dem Einsatz 15 h lang bei 40°C in 5%-iger Schwefelsäure gelagert.

Anschließend wurde ein Ansatz von 18,2% DMF, 9, 1 % Wasser, 63,6 % Methanol und 9,1% Maleinsäuredimethylester bei einer Stromstärke von 0,6 A und einer Temperatur von 28-35°C gefahren. Um die Nafionmembran aus ihrer H⁺-Form in eine Salzform (Hier: Li⁺-Form) zu überführen, wurden dem Elektrolyten 0,5 ml einer wässrigen LiOH-Lösung zugesetzt, der pH-Wert der Lösung blieb mit 7,0 bis 7,5 im Neutralbereich.

Die Elektrolyse wurde auf einen Maleinsäureumsatz von 51 % gefahren. Das GC-Flächenverhältnis Maleinsäuredimethylester:Butantetracarbonsäurester im Elektrolyseaustrag war 1,00:0,70, das GC-Flächenverhältnis Maleinsäuredimethylester:Bernsteinsäurester im Elektrolyseaustrag war 1,00:0,34; Methoxybernsteinsäureester wurde nicht in nennenswerter Menge (>3%) gebildet.

Als Anodenprodukt war N-Methoxymethyl-N-methylformamid entstanden mit einer Selektivität von 95% entstanden.

## Patentansprüche

1. Verfahren zur Herstellung von Butantetracarbonsäurederivaten als Wertprodukt I durch gekoppelte Elektrosynthese, wobei eine Verbindung ausgewählt aus der Gruppe bestehend aus Maleinsäureestern, Maleinsäureesterderivaten, Fumarsäureestern oder Fumarsäureesterderivaten, bei welchen wenigstens ein Wasserstoffatom der Positionen 2 und 3 durch inerte Gruppen substituiert sein kann, kathodisch reduziert wird und an der Anode ein Wertprodukt II erhalten wird, **dadurch gekennzeichnet, daß** als Wertprodukt II ein Produkt ausgewählt wird aus einer Gruppe bestehend aus Acetalen aromatischer Aldehyde, methoxylierten Heterocyclen, Aromaten und Olefinen, methoxylierten Amiden sowie α-Hydroxyketalen, α-Hydroxyacetalen, Carbonsäuren und Carbonsäureestern, und im Elektrodenmaterial Materialien auf Kohlenstoffbasis enthalten sind.

2. Verfahren nach Anspruch 1, **dadurch gekennzeichnet, daß** es in einer ungeteilten elektrochemischen Zelle durchgeführt wird.

3. Verfahren nach Anspruch 1 oder 2, **dadurch gekennzeichnet, daß** das Maleinsäureester- oder Fumarsäureesterderivat ausgewählt wird aus einer Gruppe bestehend aus Methyl-, Cyano-, Hydroxymethyl- sowie Methoxysubstituierten Maleinsäureester- oder Fumarsäurederivaten.

4. Verfahren nach einem der Ansprüche 1 bis 3, **dadurch gekennzeichnet, daß** das Maleinsäureester- oder Fumarsäureesterderivat Maleinsäuredimethylester bzw. Fumarsäuredimethylester ist.

5. Verfahren nach einem der Ansprüche 1 bis 4, **dadurch gekennzeichnet, daß** bei seiner Durchführung ein Lösemittel oder ein Lösemittelgemisch eingesetzt wird, wobei das Lösemittel oder das Lösemittelgemisch einen Gewichtsanteil von größer oder gleich 40 % bezogen auf das Gesamtgewicht aller bei dem Verfahren eingesetzten Substanzen aufweist.

6. Verfahren nach Anspruch 5, **dadurch gekennzeichnet, daß** das eingesetzte Lösemittel oder Lösemittelgemisch ausgewählt werden aus einer Gruppe bestehend aus Methanol, Ethanol, Essigsäure, Dimethylsulfoxid, Tetrahydrofuran, Dioxan, Acetonitril und Gemischen aus zwei oder mehr davon.

7. Verfahren nach einem der Ansprüche 1 bis 6, **dadurch gekennzeichnet, daß** bei seiner Durchführung wenigstens ein Leitsalz eingesetzt wird.

8. Verfahren nach Anspruch 7, **dadurch gekennzeichnet, daß** das wenigstens eine Leitsalz ausgewählt wird aus einer Gruppe bestehend aus Tetraalkylammoniumsalzen, Tetrafluorboraten, Alkalimetallsalzen, Salzen von aromatisch substituierten Sulfonsäuren, Salzen der Methansulfonsäure, Salzen der Perchlorsäure, Bromiden, Iodiden, Phosphaten, Phosphonaten, Alkoxycarbonaten, Carboxylaten, Sulfaten, Alkylsulfonaten sowie Alkylsulfaten.

9. Verfahren nach einem der Ansprüche 1 bis 6, **dadurch gekennzeichnet, daß** es in Gegenwart einer Ionenaustauschermembran durchgeführt wird.

## Claims

1. A process for preparing butanetetracarboxylic acid derivatives as product of value I by coproduction electrosynthesis, by cathodically reducing a compound selected from the group consisting of maleic esters, maleic ester derivatives, fumaric esters and fumaric ester derivatives where at least one hydrogen atom in positions 2 and 3 may be replaced by inert groups and obtaining a product of value II at the anode, wherein the product of value II is a product selected from the group consisting of acetals of aromatic aldehydes, methoxylated heterocycles, aromatics and olefins, orthoesters, methoxylated amides, α-hydroxyketals, α-hydroxyacetals, carboxylic acids and carboxylic esters, and the electrode material comprises materials based on carbon.

2. The process according to claim 1, conducted in an undivided electrochemical cell.

3. The process according to claim 1 or 2, wherein the maleic ester or fumaric ester derivative is selected from the group consisting of methyl-, cyano-, hydroxymethyl- or methoxy-substituted maleic ester and fumaric ester derivatives.

4. The process according to any of claims 1 to 3, wherein the maleic ester or fumaric ester derivative is dimethyl maleate or dimethyl fumarate.

5. The process according to any of claims 1 to 4, wherein a solvent or solvent mixture is used in a weight fraction of not less than 40% based on the total weight of all the substances used in the process.

6. The process according to claim 5, wherein the solvent or solvent mixture used is selected from the group consisting of methanol, ethanol, acetic acid, dimethyl sulfoxide, tetrahydrofuran, dioxane, acetonitrile and mixtures of two or more thereof.

7. The process according to any of claims 1 to 6, wherein at least one conducting salt is used.

8. The process according to claim 7, wherein the at least one conducting salt is selected from the group consisting of tetraalkylammonium salts, tetrafluoroborates, alkali metal salts, salts of aromatically substituted sulfonic acids, salts of methanesulfonic acid, salts of perchloric acid, bromides, iodides, phosphates, phosphonates, alkoxycarbonates, carboxylates, sulfates, alkylsulfonates and alkyl sulfates.

9. The process according to any of claims 1 to 6, wherein an ion exchange membrane is present.

## Revendications

1. Procédé de fabrication de dérivés d'acide butanetétracarboxylique comme produit d'intérêt I par électrosynthèse couplée, dans lequel on réduit par voie cathodique un composé choisi dans le groupe constitué des esters d'acide maléique, des dérivés d'esters d'acide maléique, des esters d'acide fumarique ou des dérivés d'esters d'acide fumarique, dans lesquels au moins un atome d'hydrogène des positions 2 et 3 peut être substitué par des groupements inertes, et dans lequel on obtient un produit d'intérêt II à l'anode, **caractérisé en ce que** le produit d'intérêt II est un produit choisi dans un groupe constitué d'acétals d'aldéhydes aromatiques, d'hétérocycles méthoxylés, de composés aromatiques et d'oléfines, d'amides méthoxylés ainsi que d'α-hydroxycétals, d'α-hydroxyacétals, d'acides carboxyliques et d'esters d'acide carboxylique et **en ce que** des matériaux à base de carbone sont contenus dans le matériau d'électrode.

2. Procédé selon la revendication 1, **caractérisé en ce qu'**il est réalisé dans une cellule électrochimique non divisée.

3. Procédé selon la revendication 1 ou 2, **caractérisé en ce que** le dérivé d'ester d'acide maléique ou d'ester d'acide fumarique est choisi dans un groupe constitué de dérivés d'ester d'acide maléique ou d'acide fumarique substitués par un groupement méthyle, cyano, hydroxyméthyle et méthoxy.

4. Procédé selon l'une quelconque des revendications 1 à 3, **caractérisé en ce que** le dérivé d'ester d'acide maléique ou d'acide fumarique est l'ester diméthylique d'acide maléique ou d'acide fumarique.

5. Procédé selon l'une quelconque des revendications 1 à 4, **caractérisé en ce que** l'on utilise un solvant ou un mélange de solvants lors de sa mise en oeuvre, le solvant ou le mélange de solvants présentant une fraction en poids supérieure ou égale à 40 % par rapport au poids total de toutes les substances utilisées dans le procédé.

6. Procédé selon la revendication 5, **caractérisé en ce que** l'on choisit le solvant ou le mélange de solvants utilisé dans un groupe constitué des substances suivantes : méthanol, éthanol, acide acétique, sulfoxyde de diméthyle, tétrahydrofuranne, dioxanne, acétonitrile et des mélanges de deux de ces substances ou plus.

7. Procédé selon l'une quelconque des revendications 1 à 6, **caractérisé en ce que** l'on utilise au moins un sel conducteur lors de sa mise en oeuvre.

8. Procédé selon la revendication 7, **caractérisé en ce que** l'on choisit ledit au moins un sel conducteur dans un groupe constitué des sels de tétraalkylammonium, des tétrafluoroborates, des sels de métaux alcalins, des sels d'acides sulfoniques à substitution aromatique, des sels d'acide méthane-sulfonique, des sels d'acide perchlorique, des bromures, des iodures, des phosphates, des phosphonates, des alcoxycarbonates, des carboxylates, des sulfates, des sulfonates d'alkyle ainsi que des sulfates d'alkyle.

9. Procédé selon l'une quelconque des revendications 1 à 6, **caractérisé en ce que** sa mise en oeuvre se fait en présence d'une membrane échangeuse d'ions.
